# EUROPÄISCHE PATENTSCHRIFT

(11) **EP 1 702 559 B1**
(45) Veröffentlichungstag und Bekanntmachung des Hinweises auf die Patenterteilung: **29.10.2008**
(21) Anmeldenummer: 06003581.3
(22) Anmeldetag: 22.02.2006
(51) Int. Cl.: A61B 5/00

(54) **Verfahren und System zur Untersuchung des Glucosestoffwechsels**
Method and system for analyzing glucose metabolism
Procédé et dispositif d'analyse du metabolism glucosique

(30) Priorität: 15.03.2005 DE 102005011755; 16.07.2005 DE 102005033358
(43) Veröffentlichungstag der Anmeldung: 20.09.2006
(73) Patentinhaber: Roche Diagnostics GmbH, 68305 Mannheim (DE); F.HOFFMANN-LA ROCHE AG, 4070 Basel (CH)
(72) Erfinder: Staib, Arnulf, Dr., 64646 Heppenheim (DE); Pill, Johannes, Dr. Dr., 69181 Leimen (DE); Kotulla, Reinhard, Dr., 67245 Lambsheim (DE); Hegger, Rainer, Dr., 61118 Bad Vilbel (DE)
(74) Vertreter: Mommer, Niels

(56) Entgegenhaltungen:
- US-B1- 6 272 480
- AGUIRRE LUIS ANTONIO ET AL.: "Nonlinear multivariable modeling and analysis of sleep apnea time series" COMPUTERS IN BIOLOGY AND MEDICINE, Bd. 29, Nr. 3, Mai 1999 (1999-05), Seiten 207-228, XP002413732

## Beschreibung

Die Erfindung betrifft ein Verfahren zur Untersuchung des Glucosestoffwechsels eines Menschen auf krankheitsrelevante und/oder krankheitsbedingte Besonderheiten, bei dem die Glucosekonzentration g(t₁) bis g(tₙ) einer Körperflüssigkeit, insbesondere Blut, zu Zeitpunkten t₁ bis tₙ, die über einen Zeitraum von mindestens vier Stunden, vorzugsweise mindestens sechs Stunden verteilt sind, gemessen wird. Die Erfindung betrifft ferner ein System zur Untersuchung des Glucosestoffwechsels eines Menschen mit einer Meßeinheit zur Messung der Glucosekonzentration einer Körperflüssigkeit, insbesondere Blut.

Eine laufende Überwachung der Blutglucosekonzentration, bei der beispielsweise im Abstand von wenigen Minuten Meßwerte gewonnen werden, ist im Stand der Technik unter dem Schlagwort "continuous monitoring", beispielsweise aus der US 5,507,288, US 6,272,480 oder der EP 1102194 A2 bekannt. Bei diesen Anwendungen geht es darum, die zur Behandlung von Diabetes erforderlichen Insulingaben zu optimalen Zeitpunkten in optimalen Mengen zu verabreichen, um den Blutzuckerspiegel eines Diabetikers ähnlich wie bei einer gesunden Person ständig innerhalb enger Grenzen zu halten.

Die Blutglucosekonzentration eines Patienten ist von größter medizinischer Bedeutung. Studien haben zu dem Ergebnis geführt, daß äußerst schwerwiegende langfristige Folgen des Diabetes Mellitus (beispielsweise Erblinden aufgrund einer Retinopathie) vermieden werden können, wenn der Blutzuckerspiegel sorgfältig überwacht und innerhalb enger Grenzen gehalten wird.

Verfahren, bei denen durch kontinuierliche Überwachung Meßwerte der Blutglucosekonzentration gewonnen werden, bieten in diesem Zusammenhang den Vorteil, daß einem Anstieg der Blutglucosekonzentration über einen kritischen Wert hinaus durch eine Insulingabe zeitnah entgegengewirkt werden kann. Insbesondere kann auf der Grundlage der Meßwerte auch eine künftige Blutglucosekonzentration über einen Zeitraum von bis zu einer halben Stunde vorhergesagt werden, so daß durch eine rechtzeitige Insulingabe ein gefährlicher Anstieg der Blutglucosekonzentration verhindert werden kann (z.B. US 6,272,480).

Im Rahmen der vorliegenden Erfindung wird ebenfalls eine große Menge von zeitlich relativ dicht aufeinanderfolgenden Meßwerten der Blutglucosekonzentration gewonnen. Im Gegensatz zu den im vorhergehenden erwähnten Verfahren befaßt sich die vorliegende Erfindung aber nicht damit, künftige Werte der Blutglucosekonzentration vorherzusagen oder Insulingaben optimal zu steuern, sondern mit der Diagnose von Störungen, insbesondere krankheitsbedingten Störungen, des Glucosestoffwechsels.

Obwohl Diabeteserkrankungen weitverbreitet sind und schwere Schäden verursachen, bereitet eine frühzeitige und zuverlässige Diagnose noch immer erhebliche Schwierigkeiten. Zwar ist bekannt, daß Übergewicht einen Risikofaktor für eine Diabeteserkrankung darstellt, doch ist eine zuverlässige Identifikation von Prä-Typ2-Diabetikern in der Regel nicht möglich.

Die Untersuchung von Personen, bei denen ein erhöhtes Risiko einer Typ2-Diabeteserkrankung vermutet wird, mit Hilfe der sogenannten Glucose-Clamp-Technik ist ein aufwendiges Verfahren zur Bestimmung einer Insulinresistenz (A. Mehnert et al., Herausgeber, Diabetologie in Klinik und Praxis, Thieme Verlag, Stuttgart 1994, S. 57 ff.) und ist daher Spezialeinrichtungen vorbehalten. Bei einem Glucose-Clamp wird die Blutglucosekonzentration eines Patienten durch eine Glucoseinfusion auf einen erhöhten Wert eingestellt (z.B. 125 mg/dl) und durch fortwährende Glucoseinfusionen möglichst konstant auf diesem Wert gehalten. Die dafür erforderliche Glucoseinfusionsrate ist ein Maß dafür, wie schnell erhöhte Blutglucosewerte durch eine erhöhte Insulinausschüttung des Körpers abgebaut werden können. Wird bei einem Glucose-Clamp nur eine geringe Glucoseinfusionsrate festgestellt, so ist dies ein Hinweis für eine Insulinresistenz, d.h. daß Insulin in seiner Wirkung deutlich eingeschränkt ist. Eine Insulinresistenz geht einer manifesten Typ2-Diabeteserkrankung oft um Jahre voraus. Wird sie rechtzeitig erkannt, kann sie durch entsprechende Änderung der Lebensgewohnheiten, wie z.B. Nahrungsmenge und -zusammensetzung, und/oder mit Insulin-Sensitizern behandelt und der Ausbruch einer Diabeteserkrankung verhindert werden.

Ein schwerwiegender Nachteil einer auf einen Glucose-Clamp gestützten Diagnose ist jedoch, daß beispielsweise adipöse Probanden zwar eine erhöhte Insulinresistenz aufweisen, diese aber bei den meisten nicht weiter ansteigt, also kein Prä-Typ2-Diabetes-Status vorliegt. In letzterem Fall wäre eine Behandlung zur Prävention eines Typ-2-Daibetes nicht indiziert, sowohl pharmakoökonomisch als auch wegen der einer jeden Pharmakotherapie immanenten Risiken.

Eine verläßliche Selektion der Hochrisikopatienten für Diabetes ist mit einem Glucose-Clamp nicht möglich. Geeignete Therapien zur Behandlung von Prä-Typ2-Diabetikern sind an sich vorhanden (Insulin-Sensitizer mit Verbesserung des Lipidprofils), jedoch fehlt es an einer geeigneten Diagnostik, um diese rechtzeitig und gezielt einsetzen zu können.

Den Bedarf einer frühzeitigen und zuverlässigen Diagnose von Prä-Typ2-Diabetes haben auch die Autoren der WO 00/65366 erkannt. Das dort vorgeschlagene Verfahren beruht auf einer einmaligen Abnahme von Blut, der anschließenden Messung des Blutglucosegehalts und einem NMR-Spektrum zur Bestimmung des Lipidprofils sowie einer abschließenden Risikoklassifizierung hinsichtlich Typ2-Diabetes durch Kombination der gemessenen Parameter. Allerdings ist das Lipidprofil durch zahlreiche transiente Faktoren bestimmt und eine mögliche Korrelation zwischen Lipidprofil und Diabetes bestenfalls evidenzgestützt, also populationsbasiert und nicht ohne weiteres auf einen individuellen Fall übertragbar. Auch mit diesem Verfahren ist deshalb eine frühzeitige Diagnose von Prä-Typ2-Diabetes nicht mit der gewünschten Zuverlässigkeit möglich.

Aufgabe der Erfindung ist es deshalb, einen Weg aufzuzeigen, wie Störungen des Glucosestoffwechsels frühzeitig erkannt werden können.

Diese Aufgabe wird gelöst durch ein Verfahren zur Untersuchung des Glucosestoffwechsels eines Menschen auf krankheitsrelevante und/oder krankheitsbedingte Besonderheiten, umfassend die folgenden Schritte: Messen der Glucosekonzentration g(t₁) bis g(tₙ) einer Körperflüssigkeit, insbesondere Blut, zu Zeitpunkten t₁ bis tₙ, die über einen Zeitraum von mindestens vier Stunden, vorzugsweise mindestens sechs Stunden verteilt sind, Ermitteln von Datenpunkten in Phasenraumkoordinaten aus den Meßwerten g (t₁) bis g (tₙ) der Glucosekonzentration, Aufbereiten der Datenpunkte zur Hervorhebung krankheitsbedingter Besonderheiten des Glucosestoffwechsels des untersuchten Menschen.

Die Aufgabe wird ferner gelöst durch ein System zur Untersuchung des Glucosestoffwechsels eines Menschen auf krankheitsbedingte Besonderheiten umfassend eine Meßeinheit, mit der die Glucosekonzentration g(ti) bis g(tₙ) einer Körperflüssigkeit, insbesondere Blut, zu Zeitpunkten t₁ bis tₙ, die über einen Zeitraum von mindestens 4 Stunden, vorzugsweise mindestens 6 Stunden, verteilt sind, gemessen wird, und eine Auswerteeinheit, mit der aus den Meßwerten g(t₁) bis g(tₙ) der Glucosekonzentration Datenpunkte in Phasenraumkoordinaten ermittelt werden, und die Datenpunkte zur Hervorhebung krankheitsbedingter Besonderheiten des Glucosestoffwechsels des untersuchten Menschen aufbereitet werden.

Ohne Beschränkung der Allgemeinheit wird im folgenden von Blutglucosekonzentration gesprochen. Da sich die Erfindung nicht auf das eigentliche Messen einer Glucosekonzentration, sondern auf das Aufbereiten von Datenpunkten bezieht, kann ebensogut die Glucosekonzentration einer beliebigen anderen Körperflüssigkeit herangezogen werden, beispielsweise interstitielle Flüssigkeit oder Augenflüssigkeit, die spektroskopisch vermessen werden kann.

Im Rahmen der Erfindung wurde erkannt, daß beginnende Anomalien des Glucosestoffwechsels sich durch eine zunehmende Störung des körpereigenen Mechanismus zur Regelung der Blutglucosekonzentration auszeichnen. Die Erfinder haben daraus geschlossen, daß die für eine Diagnose relevanten Besonderheiten der Regelungsmechanismen nicht durch eine einmalige Messung oder eine Messung über einen kurzen Zeitraum von wenigen Minuten untersucht werden können, da auf diese Weise nur eine Momentaufnahme einer komplexen Dynamik erfaßt wird.

Die Blutglucosekonzentration eines Menschen schwankt im Tagesverlauf und ist sehr stark von Nahrungsaufnahme und körperlicher Aktivität abhängig. Einem einzelnen Meßwert der Blutglucosekonzentration läßt sich deshalb häufig nicht ansehen, ob er an einem kranken oder einem gesunden Menschen gemessen wurde. Erst anhand der Dynamik des Regelungssystems lassen sich krankheitsbedingte Besonderheiten zuverlässig erkennen.

Erfindungsgemäß wird die Blutglucosekonzentration über einen Zeitraum von mindestens vier Stunden, vorzugsweise mindestens sechs Stunden gemessen, so daß typische Änderungen der Blutglucosekonzentration, wie sie im Tagesverlauf beispielsweise nach Mahlzeiten auftreten, und damit verbundene Reaktionen des körpereigenen Regelungsmechanismus erfaßt werden können.

In einem weiteren Erkenntnisschritt haben die Erfinder festgestellt, daß sich Besonderheiten des körpereigenen Regelungsmechanismus in einer herkömmlichen Darstellung, bei der die Blutglucosekonzentration über der Zeit aufgetragen wird, nur schwer erkennen lassen, jedoch in einer Phasenraumdarstellung sowohl für das menschliche Auge als auch für mathematische Auswertealgorithmen deutlich hervortreten. Ein wesentliches Merkmal der vorliegenden Erfindung besteht deshalb darin, daß aus den Meßwerten der Blutglucosekonzentration g(t₁) bis g(tₙ) Datenpunkte in Phasenraumkoordinaten ermittelt werden.

In einem Phasenraum, der in der Regelungstechnik bisweilen auch als "Zustandsraum" bezeichnet wird, läßt sich jeder mögliche Zustand eines dynamischen Systems durch einen Punkt repräsentieren. Beispielsweise können die Phasenraumkoordinaten eines bewegten Teilchens dessen Ort und Impuls oder dessen Impuls und Beschleunigung umfassen. Ein wesentliches Kennzeichen eines Phasenraums ist, daß die Zeit keine Koordinate darstellt. Die zeitlich aufeinanderfolgenden Zustände des Systems bilden eine Linie in dem Phasenraum, die als Trajektorie bezeichnet wird und deren Verlauf die Dynamik des Systems charakterisiert.

Eine Trajektorie kann in der Praxis selbstverständlich nur näherungsweise ermittelt werden, einerseits da Meßwerte stets mit unvermeidlichen Meßfehlern behaftet sind und andererseits nicht beliebig dicht ermittelt werden können. Im Sinne der Anmeldung wird unter Trajektorie deshalb eine aus den Datenpunkten ermittelte Linie im Phasenraum verstanden, welche die theoretisch exakte Trajektorie näherungsweise beschreibt.

Ein weiteres wichtiges Beispiel für Phasenraumkoordinaten sind sogenannte delay-Koordinaten. In delay-Koordinaten wird der Zustand eines dynamischen Systems nicht durch mehrere gleichzeitig gemessene Zustandsgrößen (beispielsweise Ort und Impuls eines Teilchens) charakterisiert, sondern durch mehrere Werte einer einzigen Zustandsgröße, die zu Zeitpunkten gemessen wurden, die sich voneinander um eine delay-Verzögerungszeit τ unterscheiden.

Für die vorliegende Erfindung geeignete Phasenraumkoordinaten sind beispielsweise die Blutglucosekonzentration g(t) und deren Änderungsgeschwindigkeit g'(t) oder delay-Koordinaten g(t) und g(t-τ). Falls delay-Koordinaten verwendet werden, wird für die delay-Verzögerungszeit τ am besten ein Wert zwischen 10 Minuten und 90 Minuten, bevorzugt zwischen 15 Minuten und 30 Minuten, gewählt.

Typ2-Diabetes ist eine chronische Stoffwechselkrankheit, die verschiedene Stadien durchläuft. Jedes Stadium stellt einen bestimmten pathologischen Zustand des Glucosestoffwechsels dar und macht speziell an den jeweiligen Zustand angepaßte therapeutische Maßnahmen erforderlich. Eine Typ2-Diabetes-Erkrankung beginnt mit einer Beeinträchtigung der körpereigenen Regulationen des Glucosespiegels. Diese Beeinträchtigung manifestiert sich in einer trägeren Gegenregulation bei Nahrungsaufnahme verbunden mit verringerter initialer Insulinsekretion der Bauchspeicheldrüse. In dem nächsten Stadium ist die gesamte endogene Insulinsekretion bei Nahrungsaufnahme reduziert, so daß ausgedehnte Hyperglykämien auftreten. In dem darauffolgenden Stadium kommt die endogene Insulinsekretion praktisch zum Erliegen, so daß die körpereigene Regulation des Glucosespiegels nur noch Hypoglykämien entgegenwirkt. In dem letzten Stadium der Erkrankung geht auch dieser endogene Regulationsmechanismus verloren.

Für die Behandlung dieser Stadien des Typ2-Diabetes stehen verschiedene therapeutische Maßnahmen, beispielsweise Diät, orale Medikation zur Steigerung der Insulinsensitivität und Insulin, zur Verfügung. Der gezielte Einsatz dieser therapeutischen Maßnahmen setzt ein sogenanntes Staging, d.h. eine zuverlässige Diagnose des jeweils vorliegenden Krankheitsstadiums voraus. Mit der vorliegenden Erfindung läßt sich eine zuverlässige Diagnose regelmäßig erheben, so daß im Rahmen eines Staging eine individuelle Therapieoptimierung möglich ist.

Obwohl auch existierende Diagnoseverfahren, beispielsweise die Messung der Insulinsekretion und Insulinsensitivität, zum Staging herangezogen werden können, ist der damit verbundene Aufwand prohibitiv, nicht zuletzt vor dem Hintergrund, daß die individuelle Therapieoptimierung eine möglichst regelmäßige Anpassung der Maßnahmen impliziert.

Die bei Anwendung eines erfindungsgemäßen Verfahrens gewonnenen Datenpunkte können auf unterschiedliche Art und Weise aufbereitet werden, um einem Arzt die Diagnose zu erleichtern. Verschiedene Möglichkeiten zur Aufbereitung der Datenpunkte und weitere Einzelheiten und Vorteile der Erfindung werden anhand von Ausführungsbeispielen unter Bezugnahme auf die beigefügten Zeichnungen erläutert. Die darin dargestellten Besonderheiten können einzeln oder in Kombination verwendet werden, um bevorzugte Ausgestaltungen der Erfindung zu schaffen. Es zeigen:
- Fig. 1: einen typischen Verlauf der Blutglucosekonzentration eines gesunden Probanden;
- Fig. 2: einen typischen Verlauf der Blutglucosekonzentration eines Typ1-Diabetikers;
- Fig. 3: einen typischen Verlauf der Blutglucosekonzentration eines Typ2-Diabetikers;
- Fig. 4: einen typischen Verlauf der Blutglucosekonzentration eines vermeintlich gesunden Prä-Typ2-Diabetikers;
- Fig. 5: den Verlauf der Änderungsgeschwindigkeit der in Figur 1 gezeigten Blutglucosekonzentration des gesunden Probanden;
- Fig. 6: den Verlauf der Änderungsgeschwindigkeit der in Figur 2 gezeigten Blutglucosekonzentration des Typ1-Diabetikers;
- Fig. 7: den Verlauf der Änderungsgeschwindigkeit der in Figur 3 gezeigten Blutglucosekonzentration des Typ2-Diabetikers;
- Fig. 8: den Verlauf der Änderungsgeschwindigkeit der in Figur 4 gezeigten Blutglucosekonzentration des Prä-Typ2-Diabetikers;
- Fig. 9: eine Phasenraumdarstellung von Datenpunkten, die aus den in Figur 1 gezeigten Werten der Blutglucosekonzentration des gesunden Probanden gewonnen wurden;
- Fig. 10: eine Phasenraumdarstellung von Datenpunkten, die aus den in Figur 2 gezeigten Werten der Blutglucosekonzentration des Typ1-Diabetikers gewonnen wurden;
- Fig. 11: eine Phasenraumdarstellung von Datenpunkten, die aus den in Figur 3 gezeigten Werten der Blutglucosekonzentration des Typ2-Diabetikers gewonnen wurden;
- Fig. 12: eine Phasenraumdarstellung von Datenpunkten, die aus den in Figur 4 gezeigten Werten der Blutglucosekonzentration des Prä-Typ2-Diabetikers gewonnen wurden;
- Fig. 13: eine weitere Phasenraumdarstellung, die auf den in Figur 1 gezeigten Meßwerten des gesunden Probanden beruht;
- Fig. 14: eine weitere Phasenraumdarstellung, die auf den in Figur 2 gezeigten Meßwerten des Typ1-Diabetikers beruht;
- Fig. 15: eine weitere Phasenraumdarstellung, die auf den in Figur 3 gezeigten Meßwerten des Typ2-Diabetikers beruht;
- Fig. 16: eine weitere Phasenraumdarstellung, die auf den in Figur 4 gezeigten Meßwerten des Prä-Typ2-Diabetikers beruht;
- Fig. 17: Ergebnisse einer Kreuzvorhersage für verschiedene Datensätze;
- Fig. 18: Werte eines Störungsparameters für verschiedene Datensätze;
- Fig. 19: ein Histogramm der Verteilung von Krümmungsradien von Trajektorien in einem Teilbereich des Phasenraums am Beispiel eines Typ2-Diabetikers;
- Fig. 20: ein Histogramm der Verteilung von Krümmungsradien von Trajektorien außerhalb des Teilbereichs von Figur 19 und
- Fig. 21: ein Blockdiagramm eines erfindungsgemäßen Systems zur Untersuchung des Glucosestoffwechsels.

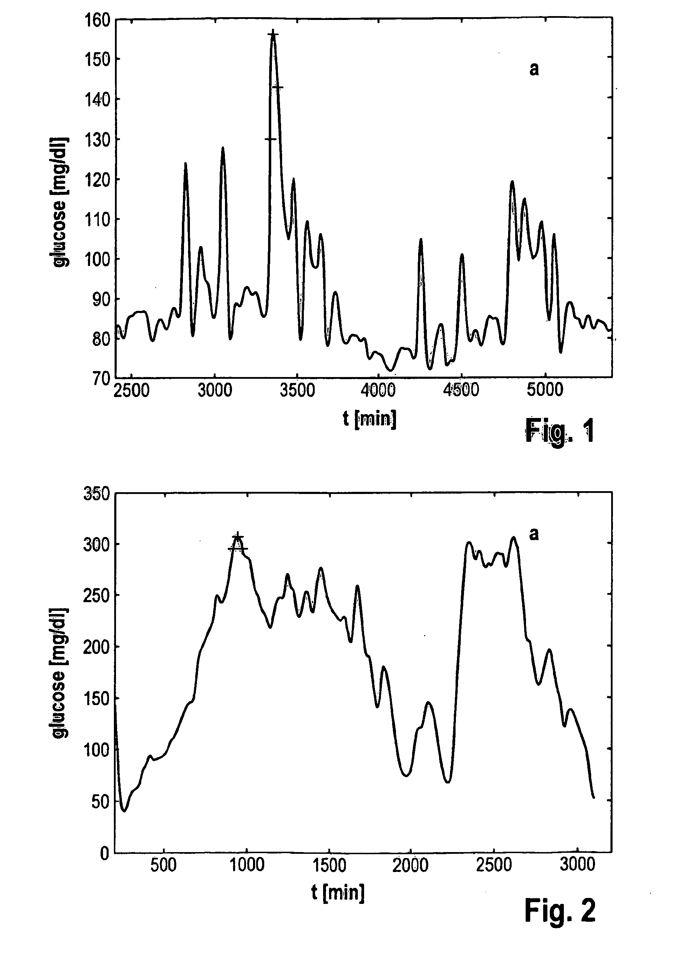

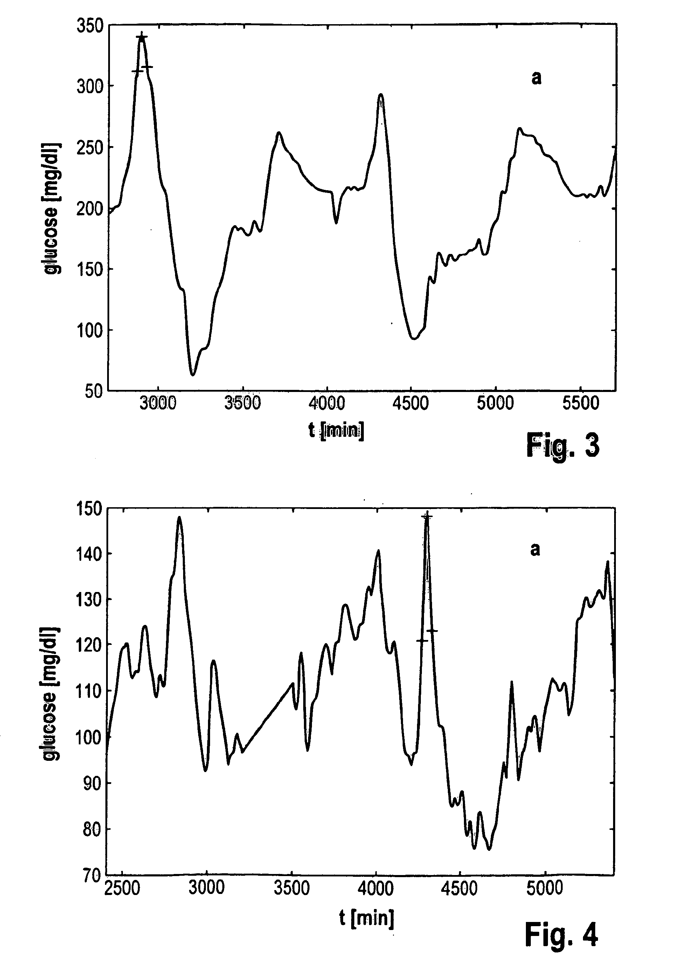

Figur 1 zeigt einen typischen Verlauf der Blutglucosekonzentration eines gesunden Probanden über einen Zeitraum von etwa 50 Stunden. Im Tagesverlauf treten infolge von Mahlzeiten oder körperlicher Anstrengung erhebliche Schwankungen der Blutglucosekonzentration g(t) auf. Erkennbar ist, daß die Blutglucosekonzentration von dem körpereigenen Regelungssystem auf einen Ziel-Wert von etwa 80 bis 90 mg/dl Glucose geregelt wird. Zum Vergleich zeigt Figur 2 einen typischen Verlauf der Blutglucosekonzentration eines Typ1-Diabetikers über einen Zeitraum von 50 Stunden. Auffällig ist das Auftreten von Konzentrationsspitzen von über 300 mg/dl Glucose und das Absinken auf Werte von weniger als 50 mg/dl. Aus diesen extrem großen Konzentrationsschwankungen läßt sich ohne weiteres auf das Vorliegen einer krankheitsbedingten Störung des Glucosestoffwechsels schließen. In Figur 3 ist ein typisches Beispiel des Blutglucosekonzentration-Verlaufs eines Typ2-Diabetikers in fortgeschrittenem Stadium der Erkrankung über der Zeit dargestellt. Ähnlich wie bei einem Typ1-Diabetiker läßt sich aus dem Auftreten von Konzentrationsspitzen von über 300 mg/dl Glucose unmittelbar auf eine krankheitsbedingte Störung des Glucosestoffwechsels schließen.

Figur 4 zeigt den Verlauf der Blutglucosekonzentration g(t) eines scheinbar gesunden Probanden, bei dem es sich in Wahrheit um einen behandlungsbedürftigen Prä-Typ2 Diabetiker handelt. Wie bei einem gesunden Probanden (siehe Fig. 1) wird die Blutglucosekonzentration von dem körpereigenen Regelungsmechanismus in einem relativ engen Band zwischen etwa 80 mg/dl und 150 mg/dl gehalten. Für Diabetes typische Konzentrationsspitzen mit Werten von 300 mg/dl Blutglucosegehalt, wie sie beispielsweise in den Figuren 2 und 3 zu sehen sind, fehlen. Es ist deshalb sehr schwer, anhand der in Figur 4 gezeigten Daten das Vorliegen von Prä-Typ2-Diabetes zu diagnostizieren.

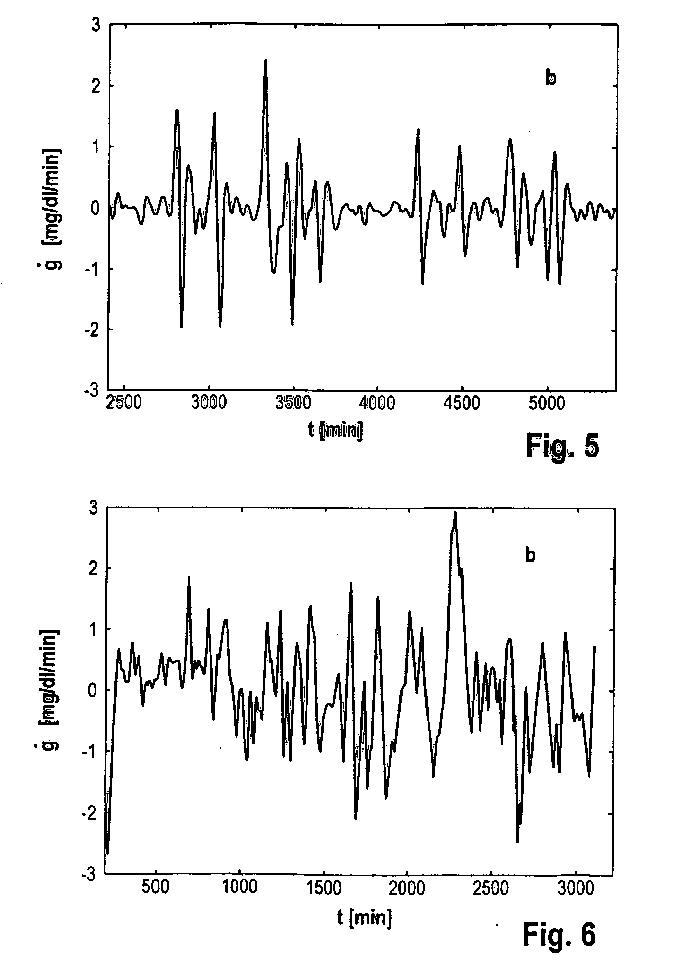

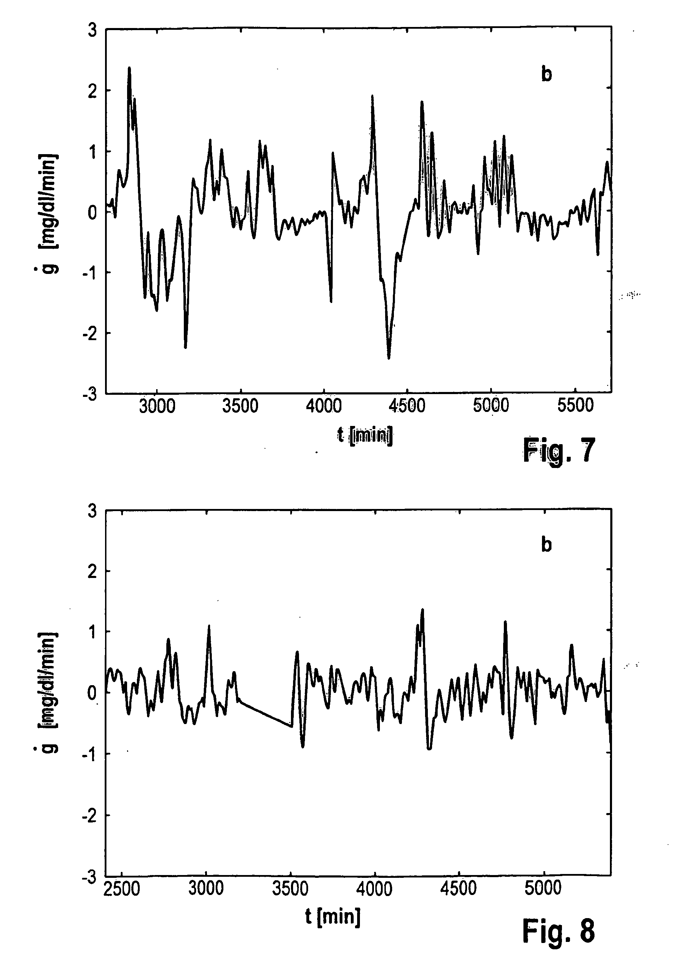

Eine weitgehend übliche Form der Datenaufbereitung besteht darin, zusätzlich zu der Meßgröße, hier also der Blutglucosekonzentration, auch deren zeitliche Ableitung über der Zeit aufzutragen. Die Figuren 5, 6, 7 und 8 zeigen jeweils die Änderungsgeschwindigkeit der Blutglucosekonzentration in mg/dl/min für die in den Figuren 1 bis 4 dargestellten Blutglucoseprofile. Dabei ist in Figur 5 die zeitliche Ableitung der in Figur 1 dargestellten Blutglucosekonzentration eines gesunden Probanden, in Figur 6 des Typ1-Diabetikers, in Figur 7 des Typ2-Diabetikers und in Figur 8 des Prä-Typ2-Diabetikers aufgetragen.

Wie man in diesen Abbildungen erkennt, schwankt die Änderungsgeschwindigkeit der Blutglucosekonzentration im Tagesverlauf derart stark, daß sich aus deren Verlauf kaum eine eindeutige Information über den Gesundheitszustand eines Probanden entnehmen läßt. Bei sämtlichen gezeigten Darstellungen, also bei gesunden Probanden ebenso wie bei Diabeteskranken, schwankt die Änderungsgeschwindigkeit der Blutglucosekonzentration im Tagesverlauf zwischen -2 und +2 mg/dl/min.

Durch eine erfindungsgemäße Datenaufbereitung lassen sich charakteristische Besonderheiten der in den im vorhergehenden beschriebenen Figuren gezeigten Daten deutlich hervorheben, so daß sich krankheitsbedingte Störungen des Glucosestoffwechsels ohne Schwierigkeit erkennen lassen. Dazu werden aus den gemessenen Blutglucosekonzentrationen zunächst Datenpunkte in Phasenraumkoordinaten ermittelt.

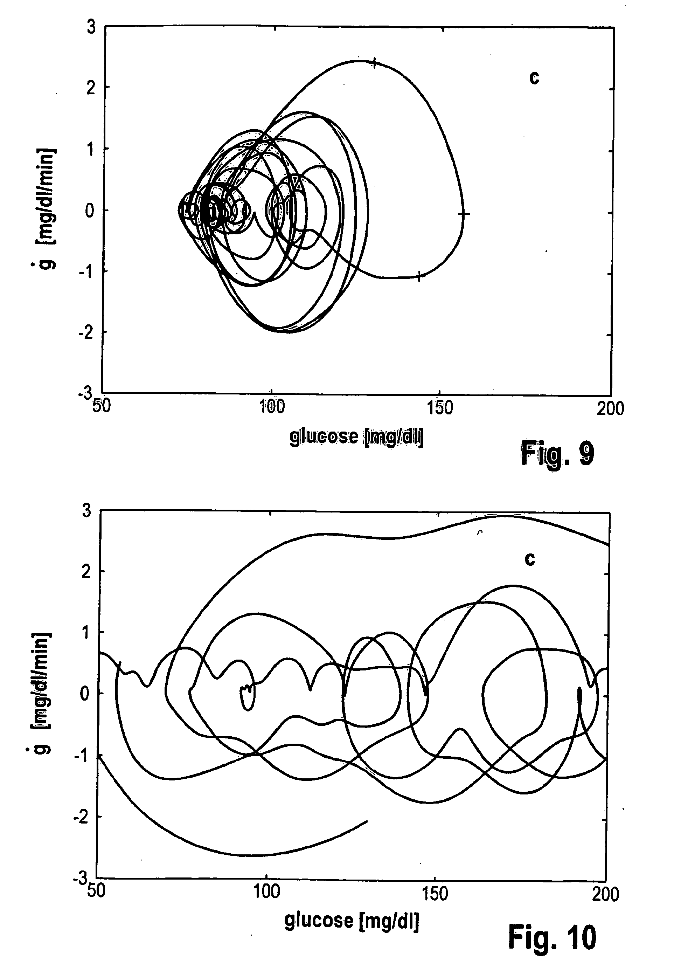

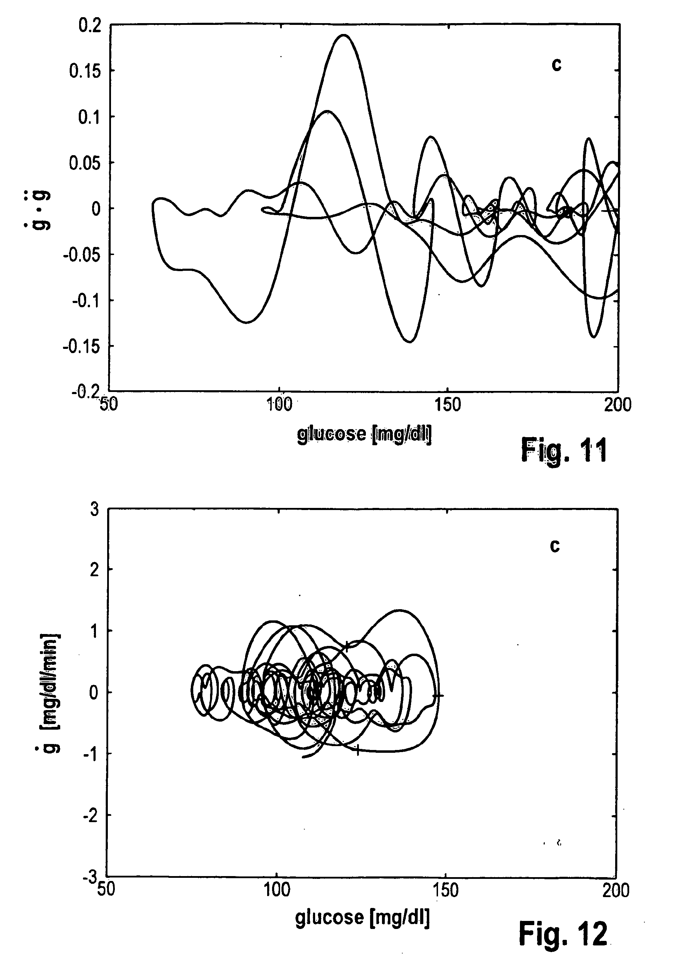

Bei den im folgenden näher erläuterten Figuren 9, 10 und 12 wurden als Phasenraumkoordinaten die Blutglucosekonzentration und deren Änderungsgeschwindigkeit zu dem jeweiligen Zeitpunkt verwendet. Für Figur 11 wurden als Phasenraumkoordinaten die Blutglucosekonzentration g(t) und das Produkt aus deren erster und zweiten Ableitung nach der Zeit verwendet. Die so ermittelten Datenpunkte wurden für die in den Figuren 1 bis 4 gezeigten Meßreihen jeweils in einer Phasenraumdarstellung aufgetragen, bei der die Änderungsgeschwindigkeit der Blutglucosekonzentration über der Blutglucosekonzentration (Fig. 9, 10 und 12) bzw. das Produkt aus der Änderungsgeschwindigkeit und der zweiten zeitlichen Ableitung der Blutglucosekonzentration über der Blutglucosekonzentration (Fig. 11) aufgetragen ist.

Die in der Figur 9 gezeigte Phasenraumdarstellung der Datenpunkte eines gesunden Probanden läßt eine Trajektorie mit einer größeren Anzahl von Schleifen erkennen, die durch einen Regelpunkt bei etwa (90 mg/dl; 0 mg/dl/min) verlaufen. In einem sehr kleinen Volumenelement um diesen Punkt bei etwa 90 mg/dl ± 3 mg/dl schneiden sich die verschiedenen Schleifen der Trajektorie, so daß dort eine erhöhte Dichte der Datenpunkte vorhanden ist.

In Figur 10 ist eine entsprechende Darstellung für einen Typ1-Diabetiker gezeigt, die auf den Daten der Figuren 2 und 6 beruht. Als wesentlicher Unterschied zu der in Figur 9 gezeigten Darstellung fällt auf, daß die Trajektorie in großen Abschnitten nur eine relativ geringe Krümmung zeigt und mehr oder weniger gleichmäßig über den dargestellten Phasenraumausschnitt verteilt ist. Insbesondere ist kein Regelungspunkt erkennbar, in dem sich mehrere Schleifen der Trajektorie schneiden.

Die in Figur 11 gezeigte Phasenraumdarstellung der Daten eines Typ2-Diabetikers ist zur in Figur 10 gezeigten Phasenraumdarstellung der Daten eines Typ1-Diabetikers dahingehend ähnlich, daß die für einen funktionierenden Regelungsmechanismus typische Schleifenform von Trajektorien, die immer wieder durch denselben Regelungspunkt hindurch laufen, fehlt. Die in Fig. 11 gezeigte Trajektorie zeigt in weiten Bereichen einen eher wellenförmigen Verlauf, bildet jedoch keine Schleifen. Insbesondere ist auch kein Teilbereich des dargestellten Phasenraumausschnitts durch eine signifikant erhöhte Dichte der Datenpunkte ausgezeichnet. Figur 11 ist zugleich ein Beispiel dafür, daß als Koordinaten auch von Zustandsgrößen (z.B. Konzentration, Änderungsgeschwindigkeit) abgeleitete Größen verwendet werden.

Vergleicht man die Phasenraumdarstellungen von Figur 9 bzw. 10, die für einen gesunden Probanden bzw. für Typ1-Diabetiker typisch sind, mit der in Figur 12 gezeigten Darstellung der Datenpunkte des scheinbar gesunden PräTyp2- Diabetikers, sind deutliche Unterschiede erkennbar.

Bei der in Figur 12 dargestellten Trajektorie eines Prä-Typ2-Diabetikers ist, insofern ähnlich wie bei dem gesunden Probanden (Fig. 9), eine Schleifenbildung erkennbar. Allerdings schneiden sich diese Schleifen nicht in einem definierten Regelungspunkt, der in Figur 9 ungefähr bei (90 mg/dl; 0 mg/dl/min) liegt, sondern sind mehr oder weniger gleichmäßig über einen Bereich zwischen (80 mg/dl; 0 mg/dl/min) und (150 mg/dl; 0 mg/dl/min) verteilt. Der für einen funktionierenden Regelungsmechanismus charakteristische Regelungspunkt ist also zu einem ausgedehnten Regelungsbereich verschmiert.

Die maximale Dichte der Datenpunkte in einem gegebenen Volumenelement ΔV = ΔxΔy der in Figur 12 gezeigten Phasenraumdarstellung mit beispielsweise Δx = 10 mg/dl und Δy = 1 mg/dl/min ist zwar wesentlich höher als bei den Phasenraumdarstellungen für Diabeteskranke (Figur 10), aber wesentlich geringer als bei einem gesunden Probanden (Fig. 9).

Die Darstellungen machen deutlich, daß ein Arzt schon mit minimalem Training anhand einer Phasenraumdarstellung von Datenpunkten des Glucoseverlaufs einen Prä-Typ2-Diabetiker, der noch keine herkömmlichen Symptome einer Diabeteserkrankung zeigt, von einem gesunden Probanden unterscheiden kann.

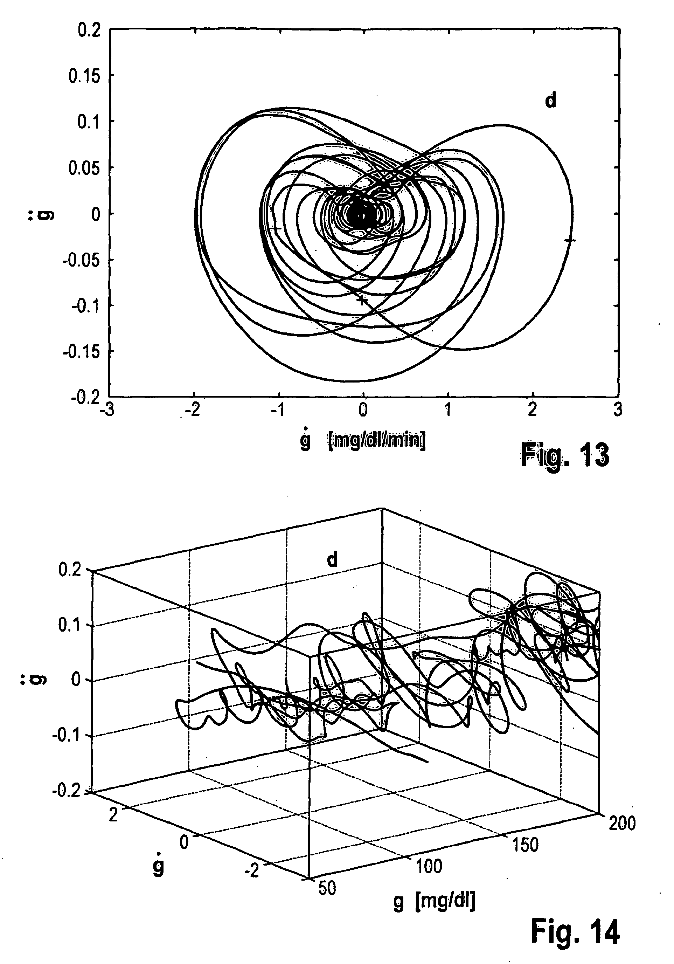

Als weiteres Beispiel einer für die vorliegende Erfindung geeigneten Phasenraumdarstellung sind in den Figuren 13 bis 16 die erste und die zweite zeitliche Ableitung der Blutglucosekonzentration (also Änderungsgeschwindigkeit und Beschleunigung) als Phasenraumkoordinaten verwendet. Im Fall von Figur 14 wurde für eine dreidimensionale Phasenraumdarstellung zusätzlich zu den beiden zeitlichen Ableitungen auch die Blutglucosekonzentration selbst als Phasenraumkoordinate verwendet.

Die in Figur 13 gezeigte Phasenraumdarstellung beruht auf den in Figur 1 gezeigten Meßwerten der Blutglucosekonzentration eines gesunden Probanden. Ebenso wie bei der in Figur 9 gezeigten Phasenraumdarstellung des gesunden Probanden zeigen sich auch in Figur 13 für einen funktionierenden Regelungsmechanismus charakteristische Schleifen. Diese Schleifen schneiden sich in einem Regelungspunkt bei etwa (0 mg/dl/min; 0 mg/dl/min²), so daß sich in einem Volumenelement ΔV = Δx mal Δy um den Regelungspunkt mit Δx = 0,2 mg/dl/min und Δy = 0,03 mg/dl/min² eine deutlich erhöhte Dichte der Datenpunkte ergibt.

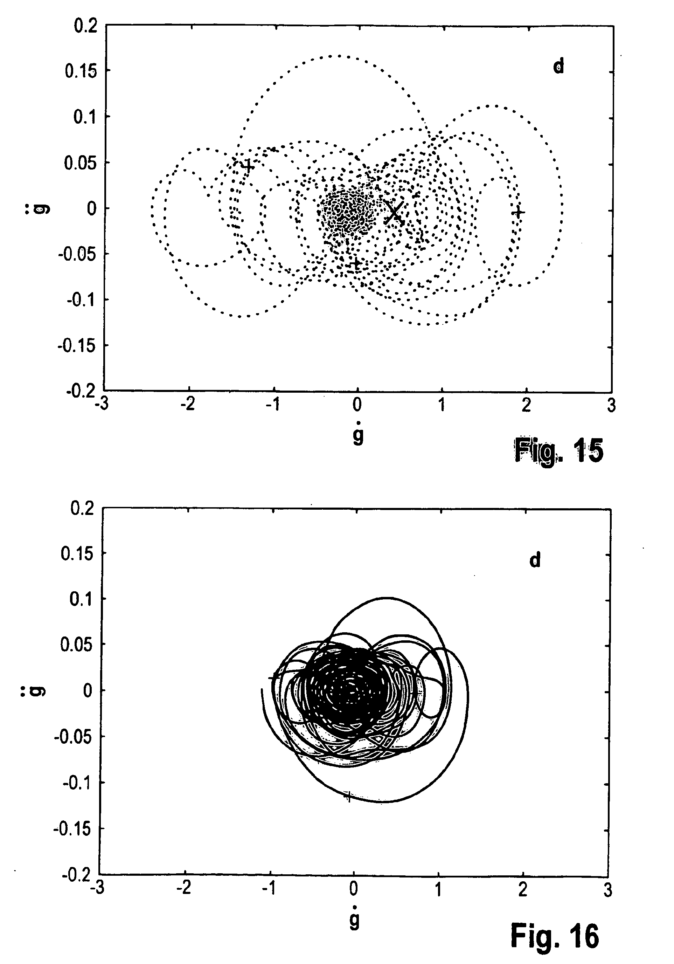

Bei den Phasenraumdarstellungen für Diabeteskranke (Figuren 14 und 15) ist keine Schleifenbildung der Trajektorie erkennbar. Ein Regelungspunkt ist in Fig. 14 gar nicht und in Fig. 15 allenfalls ansatzweise erkennbar. In Fig. 15 ist durch ein großes X zusätzlich der Schwerpunkt der Datenpunkte markiert. Bei der entsprechenden Phasenraumdarstellung des scheinbar gesunden Prä-Typ2-Diabetikers (Figur 16) ist zwar eine Schleifenbildung der Trajektorie erkennbar, jedoch ist der in Figur 14 erkennbare Volumenbereich um den Regelungspunkt mit erhöhter Datenpunktdichte zu einem Ball mit einem etwa 10mal größeren Volumen aufgebläht.

Die beschriebenen Figuren machen deutlich, daß eine Aufbereitung der Datenpunkte durch Auftragen in einer Phasenraumdarstellung erfolgen kann, die einem Arzt als Diagnosehilfe zur Verfügung gestellt werden kann. Mit bloßem Auge lassen sich in einer Phasenraumdarstellung charakteristische Besonderheiten erkennen und auf eine krankhafte Störung des Glucosestoffwechsels schließen.

Wie weiter oben erläutert wurden, werden als Koordinaten des Phasenraums Zustandsgrößen verwendet, die sich zur Beschreibung des Glucosestoffwechselssystems eignen. Für Anwendungszwecke der Erfindung bevorzugte Koordinaten sind die Glucosekonzentration und deren zeitliche Ableitungen, insbesondere als delay-Koordinaten. Unter den zeitlichen Ableitungen ist die erste zeitliche Ableitung (Änderungsgeschwindigkeit) besonders gut geeignet. Ebenfalls geeignet sind selbstverständlich Funktionen, deren Variablen von einer oder mehreren dieser primären Zustandsgrößen gebildet werden.

Ein Vergleich der Figuren 9 bis 12 mit den Figuren 13 bis 16 zeigt, daß für Gesundheits- bzw. Krankheitszustände charakteristische Besonderheiten von der Wahl der Phasenraumkoordinaten weitgehend unabhängig sind. Eine Schleifenbildung der Trajektorie und eine erhöhte Datenpunktdichte um einen Regelungspunkt sind stets Anzeichen für einen funktionierenden Regelungsmechanismus und damit für einen gesunden Glucosestoffwechsel. Mit fortschreitender Erkrankung zeigen die Trajektorien eine zunehmend geringere Schleifenbildung und die maximale Dichte der Datenpunkte um einen Regelungspunkt verringert sich, bis schließlich gar kein Regelungspunkt mehr erkennbar ist.

Wird eine Phasenraumdarstellung als Diagnosehilfe verwendet, so wird bevorzugt eine erhöhte Dichte der Datenpunkte, die für einen Regelungspunkt und damit für einen funktionierenden Regelungsmechanismus charakteristisch ist, farblich hervorgehoben.

Günstig ist es, wenn dabei die Datenpunkte in einer Farbe dargestellt werden, die von der Anzahl der Datenpunkte in einer vorgegebenen Umgebung U des jeweiligen Datenpunktes abhängt.

Gemäß einem weiteren Aspekt der vorliegenden Erfindung erfolgt die Aufbereitung der Datenpunkte dadurch, daß aus ihnen ein Störungsparameter ermittelt wird, der mit der Schwere einer Störung des Glucosestoffwechsels korreliert. Dieser Störungsparameter kann ergänzend zu einem Auftragen der Datenpunkte in einer Phasenraumdarstellung erfolgen oder diese ersetzen.

Der Störungsparameter kann beispielsweise durch eine statistische Auswertung der Datenpunkte ermittelt werden. Wie im Zusammenhang mit den gezeigten Phasenraumdarstellungen bereits erläutert wurde, ist bei einem gesunden Probanden ein relativ scharf definierter Regelungspunkt vorhanden, in dessen Umgebung eine deutlich erhöhte Dichte der Datenpunkte vorliegt. Mit Beginn einer Störung des Glucosestoffwechsels mit Krankheitswert wird dieser Regelungspunkt zunehmend unscharf, so daß die maximale Dichte der Datenpunkte entsprechend abnimmt und schließlich bei Ausbruch einer Diabeteserkrankung ganz verschwindet. Ein wichtiges Kriterium für eine statistische Auswertung ist deshalb die maximale Datenpunktdichte in dem Phasenraum. Im Rahmen einer statistischen Auswertung kann der Störungsparameter beispielsweise aus dem Anteil der Datenpunkte bestimmt werden, der in einer vorgegebenen Umgebung U eines Regelpunktes liegt, oder dem maximalen Anteil der Datenpunkte, der in einem Volumenelement gegebener Größe liegt.

Eine weitere Möglichkeit einer statistischen Auswertung besteht darin, die Hauptachsen eines Varianzellipsoids zu bestimmen und den Störungsparameter aus dem Verhältnis der Hauptachsen des Varianzellipsoids zu berechnen.

Ein Varianzellipsoid läßt sich aus der Streuung der Datenpunkte in dem Phasenraum berechnen, wobei dessen Hauptachsen die Varianz der Datenpunkte in der jeweiligen Richtung des Phasenraums angeben. Als Störungsparameter kann beispielsweise das Verhältnis der Hauptachsen einer zweidimensionalen Phasenraumdarstellung, wie sie in den Figuren 9 bis 13 gezeigt ist, verwendet werden. Sind beispielsweise L_{G} und L_{V} die Längen der skalierten (also dimensionslosen) Hauptachsen für die Varianz der Blutglucosekonzentration bzw. der Änderungsgeschwindigkeit der Blutglucosekonzentration, gilt für das Varianzellipsoid eines Prä-Typ2-Diabetikers L_{G}/L_{V} > 1, wogegen bei einem Gesunden dieser Bruch kleiner als oder gleich 1 ist.

Eine statistische Auswertung hat den Vorteil, daß eine Trajektorie nicht unbedingt erkennbar sein muß, also die Blutglucosekonzentration nicht unbedingt in so kurzen Zeitabständen gemessen werden muß, daß sich in der Phasenraumdarstellung erkennen läßt, welche Datenpunkte jeweils zeitlich aufeinander folgen. Zusammenfassend läßt sich sagen, daß bei einer statistischen Auswertung ausgenutzt wird, daß bei einem gesunden Patienten die Datenpunkte wesentlich dichter um einen Regelpunkt liegen als bei einem Diabeteskranken. Bei zunehmender Erkrankung wird der Regelungsmechanismus zur Einstellung der Blutglucosekonzentration immer schlechter, so daß sich immer häufiger Datenpunkte finden, die weit von dem Regelpunkt entfernt sind.

Eine weitere Möglichkeit zur Berechnung eines Störungsparameters ist die Auswertung einer Kreuzvorhersage eines Trajektorienverlaufs. Bei dieser Vorgehensweise wird durch Optimieren von Modellparametern eine Vorhersagefunktion erzeugt, mit welcher der Verlauf einer auszuwertenden Trajektorie in einem Bereich des Phasenraums approximiert werden kann. Im einfachsten Fall handelt es sich bei der Vorhersagefunktion um eine einfache Extrapolation eines gegebenen Trajektorienabschnitts.

Bei einer Kreuzvorhersage wird die durch Anpassen der Optimierungsparameter für eine auszuwertende Trajektorie gewonnene Vorhersagefunktion zur Vorhersage einer Referenz-Trajektorie in dem entsprechenden Bereich des Phasenraums verwendet. Beispielsweise wird auf diese Weise eine Vorhersagefunktion an eine Trajektorie der Daten eines zu untersuchenden Patienten angepaßt und die so gewonnene Funktion zur Vorhersage der Trajektorie eines gesunden Probanden und eines diabeteskranken Patienten verwendet. Das Fehlermaß der mit der so gewonnenen Vorhersagefunktion berechneten Trajektorie zu der Trajektorie des gesunden Probanden bzw. des Diabetikers gibt an, wie weit sich der Gesundheitszustand des zu untersuchenden Patienten von dem gesunden Idealzustand entfernt und an den Zustand eines Diabetikers angenähert hat.

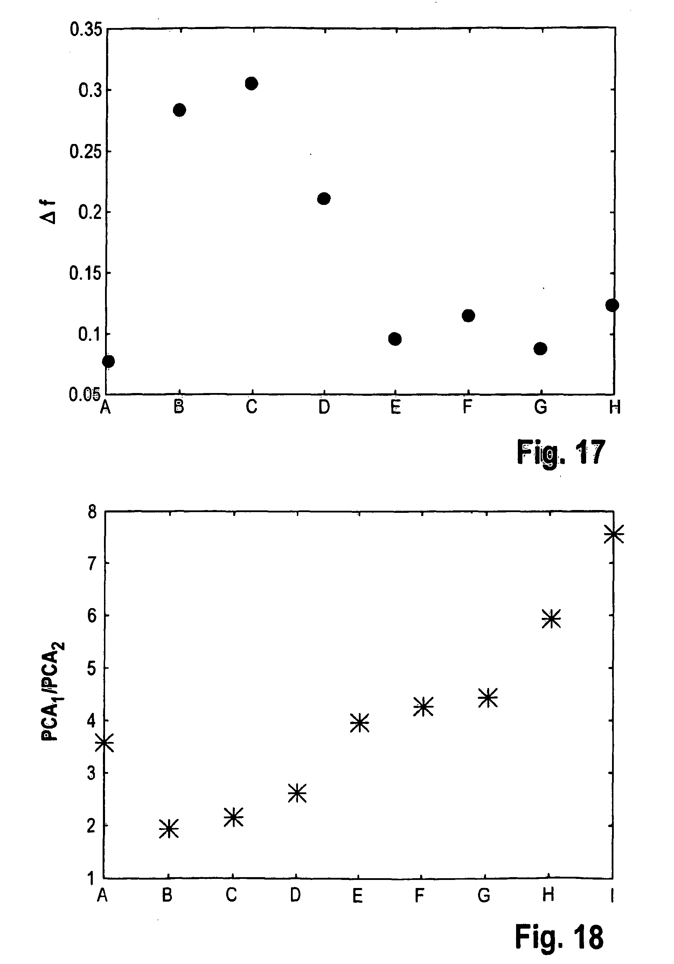

Als Beispiel für diese Vorgehensweise ist in Figur 17 der mit einer Kreuzvorhersage gewonnene Vorhersagefehler Δf für einen Testdatensatz A aufgetragen. Dabei wurden Datensätze A bis H als Referenzdatensätze verwendet. Erwartungsgemäß erhält man nur einen sehr kleinen Vorhersagefehler, wenn man den Testdatensatz A bei der Kreuzvorhersage auch als Referenzdatensatz verwendet. Werden für den Testdatensatz die Referenzdatensätze B bis H verwendet, ergeben sich in den Fällen der Datensätze E bis H etwas größere Vorhersagefehler Δf und in den Fällen der Referenzdatensätze B bis D wesentliche größere Vorhersagefehler Δf. Bei dem gezeigten Beispiel wurden die Datensätze B bis D an gesunden Probanden und die Datensätze E bis H an Typ2-Diabetikern ermittelt. Anhand der in Figur 17 gezeigten Vorhersagefehler Δf läßt sich somit für den Testdatensatz A auf das Vorliegen von Typ2-Diabetes schließen.

Eine weitere Möglichkeit zur Ermittlung eines Störungsparameters ist eine geometrische bzw. differentialgeometrische Auswertung der von den Datenpunkten in dem Phasenraum gebildeten Trajektorien. Bei einer solchen Auswertung macht man sich zunutze, daß die Blutglucosekonzentration nach Mahlzeiten oder körperlicher Anstrengung um so rascher zu ihrem Regelungspunkt zurückkehrt, je besser die Regelung der Blutglucosekonzentration funktioniert. Dies bedeutet, daß bei einem gesunden Menschen die Trajektorien wesentlich stärker gekrümmt sind als bei einem Diabetiker. Insbesondere bilden die Trajektorien bei einem gesunden Menschen näherungsweise kreisförmige Schleifen, während sie bei fortschreitender Störung der Regulation des Glucosestoffwechsels die Form von zunehmend exzentrischen Ellipsoiden annehmen. Für eine geometrische Auswertung können deshalb die einzelnen Schleifen der Trajektorie näherungsweise als Ellipsoide betrachtet werden und deren Exzentrizität als Maß für die Schwere einer Erkrankung bestimmt werden.

Ein Beispiel für einen durch geometrische Auswertung gewonnenen Störungsparameter ist in Figur 18 für Datensätze A bis I dargestellt. Als Störungsparameter wurde dabei das Verhältnis der Längen der ersten und zweiten Hauptachsen verwendet, die aus einer Hauptkomponentenzerlegung des Phasenraums gewonnen wurden. Der Datensatz A wurde an einem Prädiabetiker ermittelt, die Datensätze B bis D an gesunden Probanden, die Datensätze E bis I an Typ2-Diabetikern mit unterschiedlicher Glucosestoffwechsellage. Bei den Patienten der Datensätze E bis G ist noch eine hinreichende endogene Insulinsekretion vorhanden, bei den Patienten der Datensätze H und I nicht mehr.

Figur 18 zeigt, daß sich aus Datenpunkten in Phasenraumkoordinaten ein Störungsparameter ermitteln läßt, der nicht nur deutliche Unterschiede zwischen gesunden Probanden und Diabetikern erkennen läßt, sondern auch dazu geeignet ist, verschiedene Stadien einer Diabeteserkrankung zu identifizieren. Der Störungsparameter kann deshalb auch im Rahmen eines Stagings als Stagingparameter verwendet werden. Bei dem gezeigten Beispiel liegt der Störungsparameter für Typ2-Diabetiker im frühen Stadium der Erkrankung bei etwa 4 bis 5. In diesem frühen Stadium der Erkrankung ist noch eine hinreichende Insulinsekretion vorhanden. Mit fortschreitender Diabeteserkrankung kommt die Insulinsekretion zum Erliegen und der Störungsparameter nimmt Werte von 6 und mehr an. Ein aus dem Verhältnis der ersten und zweiten Hauptkomponente (PCA₁/PCA₂) gewonnener Störungsparameter mit einem Wert von mehr als 7 ist dabei mit dem Erlöschen der Gegenregulation bei Hypoglykämie verbunden.

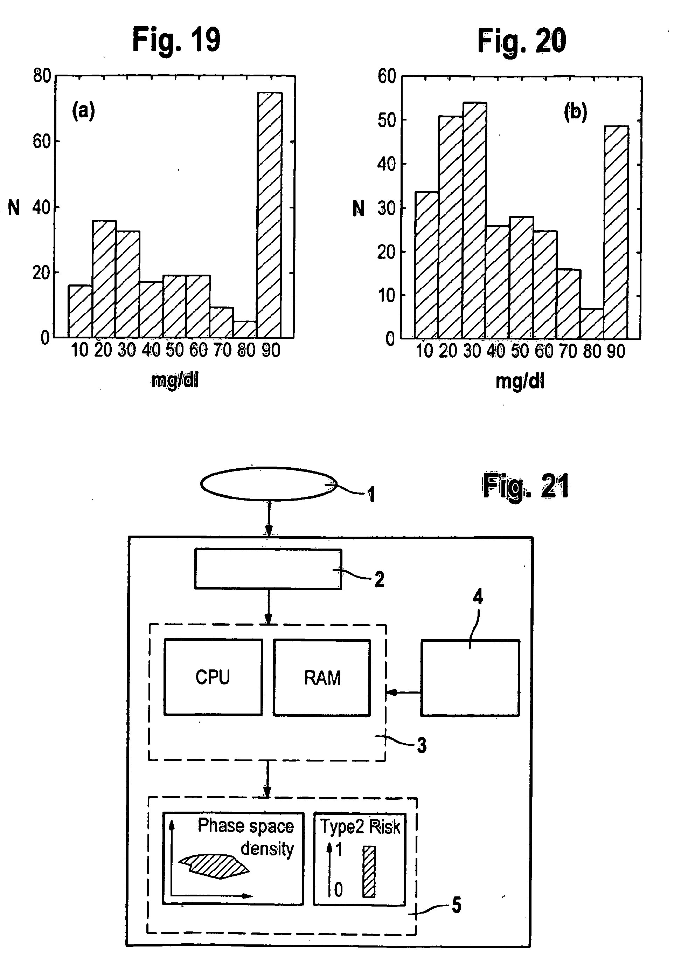

Ein weiteres Beispiel für eine differentialgeometrische Auswertung einer Phasenraumdarstellung ist in den Figuren 19 und 20 gezeigt. Diese Figuren zeigen Histogramme der Verteilung von Krümmungsradien der Trajektorien eines Typ2-Diabetikers, die in Delay-Koordinaten mit einer Delay-Zeit von fünfzehn Minuten gewonnen wurden. Über dem Krümmungsradius in mg/dl ist jeweils die Anzahl der Schleifen mit dem entsprechenden Krümmungsradius aufgetragen. Das in Figur 20 gezeigte Histogramm bezieht sich auf einen Teilbereich des Phasenraums, den die Trajektorien bei raschen Änderungen der Blutglucosekonzentration nach einer Mahlzeit einnehmen. In Figur 19 ist für denselben Patienten in entsprechender Weise ein Histogramm der Häufigkeitsverteilung von Krümmungsradien von Trajektorien in einem zweiten Teil des Phasenraums gezeigt, den die Trajektorien bei langsamen Änderungen der Blutglucosekonzentration, z.B. einige Stunden nach einer Mahlzeit, einnehmen.

Bei einer Phasenraumdarstellung in Delay-Koordinaten treten Datenpunkte, die mit einer raschen Änderung der Blutglucosekonzentration einhergehen, in einem größeren Abstand zu der Hauptdiagonalen auf. Bei dem in den Figuren 19 und 20 gezeigten Beispiel wurden Trajektorien, deren Datenpunkte mehr als 5 mg/dl von der Hauptdiagonalen entfernt waren, einer ersten, schnellen Phase der Insulinsekretion zugeordnet, auf die sich Figur 19 bezieht. In entsprechender Weise sind in Figur 20 Ergebnisse für Trajektorien gezeigt, deren Datenpunkte weniger als 5 mg/dl von der Hauptdiagonalen entfernt sind. Auf diese Weise können verschiedene Mechanismen des körpereigenen Regelungssystems untersucht werden. In einer ersten Phase der Insulinsekretion wird als Reaktion auf eine Nahrungsaufnahme sehr rasch Insulin ausgeschüttet. An die erste Phase der Insulinsekretion schließt eine zweite, langsamere Phase der Insulinsekretion an. Anhand von Figur 20 kann man also erkennen, wie gut das körpereigene Regelungssystem auf einen schnellen Anstieg von Blutglucosewerten reagiert und die erste Phase der Insulinsekretion funktioniert. In entsprechender Weise zeigt Figur 19 wie gut der langsame Regelungsmechanismus als die zweite Phase der Insulinsekretion funktioniert.

Je enger der Krümmungsradius der Trajektorien ist, desto schneller wird die Blutglucosekonzentration von dem körpereigenen Regelungsmechanismus wieder zu ihrem Sollwert zurückgeführt. Mit zunehmendem Fortschreiten einer Diabeteserkrankung treten in einem Histogramm, wie es in Figuren 19 und 20 dargestellt ist, größere Krümmungsradien der Trajektorien mit immer größeren Häufigkeiten auf, so daß aus einem Histogramm auch ein Störungsparameter für ein Staging abgeleitet werden kann.

Anhand von Figur 19 kann man erkennen, daß bei dem untersuchten Typ2-Diabetiker die zweite Phase der Insulinsekretion, also der langsame Regelungsmechanismus, bereits stark beeinträchtigt ist, was sich in einer überhöhten Häufigkeit von Trajektorien mit einem großen Krümmungsradius zeigt. Die erste Phase der Insulinsekretion, also der schnelle Regelungsmechanismus, zeigt eine wesentlich geringere Beeinträchtigung, was sich daran erkennen läßt, daß in Figur 20 Trajektorien mit geringem Krümmungsradius insgesamt häufiger auftreten, als Trajektorien mit sehr großem Krümmungsradius. Daraus kann abgeleitet werden, daß bei dem untersuchten Typ2-Diabetiker das körpereigene Regelungssystem (noch) teilweise erhalten ist, was im Rahmen eines Staging eine angepaßte Therapie ermöglicht.

Figur 21 zeigt ein Blockdiagramm eines erfindungsgemäßen Systems zur Untersuchung des Glucosestoffwechsels. Das System umfaßt eine Meßeinheit 1, mit der die Blutglucosekonzentration g(t₁) bis g(tₙ) zu Zeitpunkten t₁ bis tₙ, die über einen Zeitraum von mindestens vier Stunden, vorzugsweise mindestens sechs Stunden, verteilt sind, gemessen wird. Bei der Meßeinheit 1 handelt es sich vorzugsweise um einen implantierten Sensor, mit dem die Blutglucosekonzentration beispielsweise spektroskopisch gemessen werden kann. Die Meßwerte werden, bevorzugt drahtlos, über eine Empfangseinheit 2 an eine Auswerteeinheit 3 übermittelt. Kernstück der Auswerteeinheit sind ein Mikroprozessor (CPU) und ein elektronischer Speicher (RAM).

Mit der Auswerteeinheit 3 werden aus den gemessenen Blutglucosekonzentrationen g(t₁) bis g(tₙ) Datenpunkte in Phasenraumkoordinaten ermittelt. Diese werden in der im vorhergehenden beschriebenen Weise aufbereitet, einerseits indem sie mittels einer Anzeigeeinheit 5 in einer Phasenraumdarstellung dargestellt werden, und andererseits indem aus den Datenpunkten ein Störungsparameter ermittelt wird, der mit der Schwere einer Störung des Glucosestoffwechsels korreliert. Dieser Störungsparameter wird ebenfalls von der Anzeigeeinheit 5 angezeigt. Bei dem dargestellten Ausführungsbeispiel hat der Störungsparameter einen Wert zwischen 0 und 1. Durch den Störungsparameter wird angegeben, welches Stadium einer Glucosestoffwechselerkrankung wahrscheinlich vorliegt. Anhand des Störungsparameters kann also nicht nur eine Entscheidung darüber getroffen werden, ob eine Diabeteserkrankung vorliegt, sondern auch, in welchem Erkrankungsstadium sich der Patient befindet. Auf diese Weise wird durch das System eine optimale Therapie ermöglicht, die auf die individuellen Bedürfnisse des jeweiligen Patienten abgestimmt ist. Bevorzugt umfaßt ein erfindungsgemäßes System ferner eine Datenbank, mit der für einen gegebenen Störungsparameter eine Therapieempfehlung ermittelt und dem Arzt angezeigt wird. Das System umfaßt zusätzlich eine Eingabeeinheit 4, die beispielsweise zur Eingabe von Steuerungsbefehlen im Rahmen einer Wartung genutzt werden kann.

Bevorzugt enthält das System ferner ein Implantat (nicht gezeigt) mit einem Vorratsbehälter für Insulin und einer Vorrichtung, z.B. eine Mikropumpe zur kontrollierten Abgabe von Insulin in den Blutkreislauf eines Patienten, also eine künstliche Bauchspeicheldrüse. Mit dem erfindungsgemäßen System kann die Funktionsweise dieses Implantats überwacht und optimiert werden, indem ein Warnsignal ausgelöst wird, sobald der ermittelte Störungsparameter um mehr als eine vorgegebenen Toleranz von einem vorgegebenen Sollwert, der bei dem gezeigten Ausführungsbeispiel ein Störungsparameter mit dem Wert 0 ist, abweicht.

## Patentansprüche

1. Verfahren zur Untersuchung des Glucosestoffwechsels eines Menschen auf krankheitsrelevante und/oder krankheitsbedingte Besonderheiten, umfassend die folgenden Schritte:
Messen der Glucosekonzentration g(t₁) bis g(tₙ) einer Körperflüssigkeit, insbesondere Blut, zu Zeitpunkten t₁ bis tₙ, die über einen Zeitraum von mindestens vier Stunden, vorzugsweise mindestens sechs Stunden, verteilt sind,
Ermitteln von Datenpunkten in Phasenraumkoordinaten aus den Meßwerten g(t₁) bis g(tₙ) der Glucosekonzentration,
Aufbereiten der Datenpunkte zur Hervorhebung krankheitsbedingter Besonderheiten des Glucosestoffwechsels des untersuchten Menschen.

2. Verfahren nach Anspruch 1, **dadurch gekennzeichnet, daß** die Zeitpunkte t₁ bis tₙ in Zeitabständen von weniger als 20 Minuten, vorzugsweise weniger als 10 Minuten, besonders bevorzugt weniger als 5 Minuten, aufeinanderfolgen.

3. Verfahren nach Anspruch 1 oder 2 **dadurch gekennzeichnet, daß** ein zweidimensionales Phasenraumkoordinatensystem verwendet wird.

4. Verfahren nach einem der vorhergehenden Ansprüche, **dadurch gekennzeichnet, daß** das Aufbereiten der Datenpunkte durch Auftragen in einer graphischen Phasenraumdarstellung erfolgt, die einem Arzt als Diagnosehilfe zur Verfügung gestellt wird.

5. Verfahren nach Anspruch 4, **dadurch gekennzeichnet, daß** in der graphischen Phasenraumdarstellung eine erhöhte Dichte der Datenpunkte farblich hervorgehoben wird.

6. Verfahren nach Anspruch 5, **dadurch gekennzeichnet, daß** die Datenpunkte jeweils in einer Farbe dargestellt werden, die von der Anzahl der Datenpunkte in einer vorgegebenen Umgebung U des jeweiligen Datenpunktes abhängt.

7. Verfahren nach einem der Ansprüche 4 bis 6, **dadurch gekennzeichnet, daß** für die Darstellung der Datenpunkte in Bereichen einer erhöhten Datenpunktdichte andere Symbole verwenden werden, als in Bereichen geringerer Dichte.

8. Verfahren nach einem der Ansprüche 4 bis 7, **dadurch gekennzeichnet, daß** eine von den Datenpunkten beschriebene Trajektorie dargestellt wird.

9. Verfahren nach einem der vorhergehenden Ansprüche, **dadurch gekennzeichnet, daß** als Phasenraumkoordinaten eine Funktion der Glucosekonzentration und/oder mindestens eine ihrer zeitlichen Ableitungen verwendet werden.

10. Verfahren nach Anspruch 9, **dadurch gekennzeichnet, daß** eine der Phasenraumkoordinaten die Glucosekonzentration ist.

11. Verfahren nach Anspruch 9 oder 10, **dadurch gekennzeichnet, daß** eine der Phasenraumkoordinaten die Änderungsgeschwindigkeit der Glucosekonzentration ist.

12. Verfahren nach einem der Ansprüche 10 bis 11, **dadurch gekennzeichnet, daß** als Phasenraumkoordinaten delay-Koordinaten verwendet werden.

13. Verfahren nach Anspruch 12, **dadurch gekennzeichnet, daß** für die delay-Koordinaten eine Verzögerungszeit zwischen 10 Minuten und 90 Minuten, bevorzugt zwischen 15 Minuten und 30 Minuten, gewählt wird.

14. Auswerteeinheit, die dafür eingerichtet ist, im Betrieb aus Messwerten g(t₁) bis g(tₙ) der Glucosekonzentration einer Körperflüssigkeit zu Zeitpunkten t₁ bis tₙ, die über einen Zeitraum von mindestens 4 Stunden, vorzugsweise mindestens 6 Stunden, verteilt sind, Datenpunkte in Phasenraumkoordinaten zu ermitteln und die Datenpunkte zur Hervorhebung krankheitsbedingter und/oder krankheitsrelevanter Besonderheiten des Glucosestoffwechsels des untersuchten Menschen aufzubereiten.

15. Auswerteeinheit nach Anspruch 14, **dadurch gekennzeichnet, daß** das Aufbereiten der Datenpunkte einen Schritt einschließt, bei dem aus den Datenpunkten ein Störungsparameter ermittelt wird, der mit der Schwere einer Störung des Glucosestoffwechsels korreliert.

16. Auswerteeinheit nach Anspruch 15, **dadurch gekennzeichnet, daß** der Störungsparameter durch eine statistische Auswertung der Datenpunkte ermittelt wird.

17. Auswerteeinheit nach Anspruch 16, **dadurch gekennzeichnet, daß** der Störungsparameter aus dem Anteil der Datenpunkte bestimmt wird, der in einer vorgegebenen Umgebung U eines Regelpunktes liegt.

18. Auswerteeinheit nach Anspruch 16, **dadurch gekennzeichnet, daß** der Störungsparameter aus dem Verhältnis der Hauptachsen eines Varianzellipsoids der Datenpunkte ermittelt wird.

19. Auswerteeinheit nach Anspruch 15, **dadurch gekennzeichnet, daß** zur Ermittlung des Störungsparameters eine durch eine Folge von Datenpunkten beschriebene Trajektorie im Phasenraum ausgewertet wird.

20. Auswerteeinheit nach Anspruch 19, **dadurch gekennzeichnet, daß** der Störungsparameter aus einer Krümmung einer Schleife der Trajektorie, vorzugsweise aus den Krümmungen mehrerer Schleifen, bestimmt wird.

21. Auswerteeinheit nach Anspruch 20, **dadurch gekennzeichnet, daß** eine Schleife der Trajektorie durch eine Ellipse angenähert wird und der Störungsparameter aus dem Verhältnis der Hauptachsen der Ellipse bestimmt wird.

22. Auswerteeinheit nach Anspruch 15, **dadurch gekennzeichnet, daß** der Störungsparameter durch eine Kreuzvorhersage bestimmt wird.

23. Auswerteeinheit nach einem der Ansprüche 15 bis 22, **dadurch gekennzeichnet, daß** der Störungsparameter mit der Wahrscheinlichkeit einer Diabeteserkrankung korreliert.

24. Auswerteeinheit nach einem der Ansprüche 15 bis 23, **dadurch gekennzeichnet, daß** durch den Störungsparameter angegeben wird, welches Stadium einer Glucosestoffwechselerkrankung wahrscheinlich vorliegt.

25. System zur Untersuchung des Glucosestoffwechsels eines Menschen auf krankheitsrelevante und/oder krankheitsbedingte Besonderheiten, umfassend:
eine Meßeinheit (1), mit der die Glucosekonzentration g(t₁) bis g(tₙ) einer Körperflüssigkeit, insbesondere Blut, zu Zeitpunkten t₁ bis tₙ, die über einen Zeitraum von mindestens 4 Stunden, vorzugsweise mindestens 6 Stunden, verteilt sind, gemessen wird, und
eine Auswerteeinheit (3) nach einem der Ansprüche 14 bis 24.

26. System nach Anspruch 25, **dadurch gekennzeichnet, daß** es eine Anzeigeeinrichtung (5) zum Anzeigen der ermittelten Datenpunkte in einer Phasenraumdarstellung umfaßt.

27. System nach Anspruch 25 oder 26, **dadurch gekennzeichnet, daß** mit dem Störungsparameter unter Verwendung von in einer Datenbank gespeicherten Therapiedaten eine Therapieempfehlung erstellt und ausgegeben wird.

28. System nach einem der Ansprüche 25 bis 27, **dadurch gekennzeichnet, daß** es ein Implantat mit einem Vorratsbehälter für Insulin und eine Mikropumpe zur kontrollierten Abgabe des Insulins in den Blutkreislauf eines Patienten umfaßt, wobei von der Auswerteeinheit (3) ein Warnsignal ausgelöst wird, sobald der Störungsparameter um mehr als eine vorgegebene Toleranz von einem vorgegebenen Sollwert abweicht.

## Claims

1. Method for investigating the glucose metabolism of a human being for disease-relevant and/or disease-related particularities, comprising the following steps:
measuring the glucose concentration g(t1) to g(tn) of a body fluid, in particular blood, at time points t1 to tn that are distributed over a period of at least four hours, preferably at least six hours,
determining data points in phase space coordinates from the glucose concentration measuring values g(t1) to g(tn),
processing the data points to highlight disease-related particularities of the glucose metabolism of the investigated human being.

2. Method according to claim 1, **characterized in that** the time points t1 to tn are consecutive with time intervals of less than 20 minutes, preferably less than 10 minutes, particularly preferably less than 5 minutes.

3. Method according to claim 1 or 2 **characterized in that** a two-dimensional phase space coordinate system is used.

4. Method according to any one of the preceding claims, **characterized in that** the data points are processed by plotting in a graphic phase space representation that is provided to a physician as a diagnostic aid.

5. Method according to claim 4, **characterized in that** an increased density of data points in the graphic phase space representation is highlighted by coloring.

6. Method according to claim 5, **characterized in that** the data points are shown in a color that depends on the number of data points that are present in a pre-determined vicinity U of the respective data point.

7. Method according to any one of the claims 4 to 6, **characterized in that** different symbols are used in areas of increased data point density as compared to regions of lower density.

8. Method according to any one of the claims 4 to 7, **characterized in that** a trajectory described by the data points is displayed.

9. Method according to any one of the preceding claims, **characterized in that** a function of the blood glucose concentration and/or at least one of its derivatives with respect to time are used as phase space coordinates.

10. Method according to claim 9, **characterized in that** the glucose concentration is one of the phase space coordinates.

11. Method according to claim 9 or 10, **characterized in that** the rate of change of the glucose concentration is one of the phase space coordinates.

12. Method according to any one of the claims 10 to 11, **characterized in that** delay coordinates are used as phase space coordinates.

13. Method according to claim 12, **characterized in that** a delay time of between 10 minutes and 90 minutes, preferably between 15 minutes and 30 minutes, is selected for the delay coordinates.

14. Analytical unit configured to determine data points in phase space coordinates from measuring values g(t1) to g(tn) of the glucose concentration of a body fluid at time points t1 to tn that are distributed over a period of at least four hours, preferably at least six hours, and to process the data points in order to highlight disease-related and/or disease-relevant particularities of the glucose metabolism of the investigated human being.

15. Analytical unit according to claim 14, **characterized in that** the processing of the data points includes a step, in which the data points are used to determine a disturbance parameter that is correlated to the severity of a disturbance of glucose metabolism.

16. Analytical unit according to claim 15, **characterized in that** the disturbance parameter is determined by a statistical analysis of the data points.

17. Analytical unit according to claim 16, **characterized in that** the disturbance parameter is determined from the fraction of data points situated within a pre-determined vicinity U of a regulation point.

18. Analytical unit according to claim 16, **characterized in that** the disturbance parameter is determined from the ratio of the main axes of an ellipsoid of variance of the data points.

19. Analytical unit according to claim 15, **characterized in that** a trajectory in phase space described by a sequence of data points is analyzed in order to determine the disturbance parameter.

20. Analytical unit according to claim 19, **characterized in that** the disturbance parameter is determined from a curvature of a loop of the trajectory, preferably from the curvatures of multiple loops.

21. Analytical unit according to claim 20, **characterized in that** a loop of the trajectory is approximated by an ellipse and the disturbance parameter is determined from the ratio of the main axes of the ellipse.

22. Analytical unit according to claim 15, **characterized in that** the disturbance parameter is determined by means of a cross-prediction.

23. Analytical unit according to any one of the claims 15 to 22, **characterized in that** the disturbance parameter is correlated to the probability of a diabetic disease.

24. Analytical unit according to any one of the claims 15 to 23, **characterized in that** the disturbance parameter indicates which stage of a disease of glucose metabolism is likely to be manifest.

25. System for investigating the glucose metabolism of a human being for disease-relevant and/or disease-related particularities, comprising:
a measuring unit (1) that is used to measure the blood glucose concentration g(t1) to g(tn) at time points t1 to tn that are distributed over a period of at least 4 hours, preferably at least 6 hours, and
an analytical unit (3) according to any one of claims 14 to 24.

26. System according to claim 25, **characterized in that** it comprises a display facility (5) for displaying the data points thus determined in a phase space representation.

27. System according to claim 25 or 26, **characterized in that** the disturbance parameter is used in conjunction with therapy data stored in a database to prepare and output a therapy recommendation.

28. System according to any one of the claims 25 to 27, **characterized in that** it comprises an implant with a storage reservoir for insulin and a micropump for the controlled release of insulin into the bloodstream of a patient, whereby the analytical unit (3) triggers an alarm signal as soon as the disturbance parameter deviates by more than a pre-determined tolerance from a pre-determined nominal value.

## Revendications

1. Procédé destiné à l'étude du métabolisme du glucose d'un être humain concernant les particularités se rapportant à la maladie et/ou conditionnées par la maladie, comprenant les étapes suivantes :
► la mesure de la concentration de glucose g(t₁) à g(tₙ) d'un liquide corporel, en particulier le sang, aux instants t₁ à tₙ, qui sont répartis sur une période d'au moins quatre heures, de préférence, d'au moins six heures,
► la détermination de points de données dans les coordonnées de l'espace de phase, à partir des valeurs de mesures g(t₁) à g(tₙ) de la concentration de glucose,
► la préparation des points de données pour mettre en évidence les particularités conditionnées par le métabolisme du glucose de la personne étudiée.

2. Procédé selon la revendication 1, **caractérisé en ce que** les instants t₁ à tₙ se succèdent sur des intervalles de temps inférieurs à 20 minutes, de préférence, inférieurs à 10 minutes, de manière particulièrement préférée inférieurs à 5 minutes.

3. Procédé selon la revendication 1 ou 2, **caractérisé en ce que** l'on utilise un système de coordonnées dans l'espace de phase bidimensionnel.

4. Procédé selon l'une quelconque des revendications précédentes, **caractérisé en ce que** la préparation des points de données se fait en les reportant dans une représentation graphique de l'espace de phase, qui est mise à la disposition d'un médecin en tant qu'aide au diagnostic.

5. Procédé selon la revendication 4, **caractérisé en ce que**, dans la représentation graphique de l'espace de phase, on met en évidence une densité plus élevée des points de données à l'aide d'une couleur.

6. Procédé selon la revendication 5, **caractérisé en ce que** les points de données sont représentés respectivement par une couleur, qui dépend du nombre des points de données dans un environnement prédéterminé U du point de donnée correspondant.

7. Procédé selon l'une quelconque des revendications 4 à 6, **caractérisé en ce que,** pour la représentation des points de données dans les domaines d'une densité de points de données plus élevée, on utilise d'autres symboles que ceux utilisés dans les domaines de densité plus faible.

8. Procédé selon l'une quelconque des revendications 4 à 7, **caractérisé en ce que** l'on représente une trajectoire décrite par les points de données.

9. Procédé selon l'une quelconque des revendications précédentes, **caractérisé en ce que** l'on utilise, en tant que coordonnées de l'espace de phase, une fonction de la concentration de glucose et/ou au moins une de ses dérivées dans le temps.

10. Procédé selon la revendication 9, **caractérisé en ce que** l'une des coordonnées de l'espace de phase est la concentration de glucose.

11. Procédé selon la revendication 9 ou 10, **caractérisé en ce que** l'une des coordonnées de l'espace de phase est la vitesse de modification de la concentration de glucose.

12. Procédé selon l'une quelconque des revendications 10 à 11, **caractérisé en ce que** l'on utilise, en tant que coordonnées de l'espace de phase, des coordonnées de temps de retard.

13. Procédé selon la revendication 12, **caractérisé en ce que,** pour les coordonnées de temps de retard, on choisit un temps de retard compris entre 10 minutes et 90 minutes, de préférence, entre 15 minutes et 30 minutes.

14. Unité d'évaluation, qui est agencée pour déterminer, en cours du fonctionnement, à partir des valeurs de mesure g (t₁) à g (tₙ) de la concentration de glucose d'un fluide corporel aux instants t₁ à tₙ, qui sont répartis sur une période d'au moins 4 heurs, de préférence, d'au moins 6 heures, des points de données dans les coordonnées de l'espace de phase et pour traiter les points de données en vue de la mise en évidence de particularités conditionnées par la maladie et/ou se rapportant à la maladie du métabolisme du glucose de la personne étudiée.

15. Unité d'évaluation selon la revendication 14, **caractérisée en ce que** la préparation des points de données inclut une étape lors de laquelle on détermine à partir des points de données un paramètre de perturbations, qui établit une corrélation avec la sévérité d'une perturbation du métabolisme du glucose.

16. Unité d'évaluation selon la revendication 15, **caractérisée en ce que** le paramètre de perturbations est déterminé par une évaluation statistique des points de données.

17. Unité d'évaluation selon la revendication 16, **caractérisée en ce que** le paramètre de perturbations est déterminé à partir de la proportion des points de données, qui se situe dans un environnement prédéterminé U d'un point de réglage.

18. Unité d'évaluation selon la revendication 16, **caractérisée en ce que** le paramètre de perturbations est déterminé à partir du rapport des axes principaux d'un ellipsoïde de variance des points de données.

19. Unité d'évaluation selon la revendication 15, **caractérisée en ce qu'**en vue de la détermination du paramètre de perturbations, on évalue dans l'espace de phase une trajectoire décrite par une séquence de points de données.

20. Unité d'évaluation selon la revendication 19, **caractérisée en ce que** le paramètre de perturbations est déterminé à partir d'une courbure d'une boucle de la trajectoire, de préférence, à partir des courbures de plusieurs boucles.

21. Unité d'évaluation selon la revendication 20, **caractérisée en ce qu'**une boucle de la trajectoire est approximée par une ellipse et **en ce que** le paramètre de perturbations est déterminé à partir du rapport des axes principaux de l'ellipse.

22. Unité d'évaluation selon la revendication 15, **caractérisée en ce que** le paramètre de perturbations est déterminé par une prévision en croix.

23. Unité d'évaluation selon l'une quelconque des revendications 15 à 22, **caractérisée en ce que** le paramètre de perturbations présente une corrélation avec la probabilité d'une maladie du diabète.

24. Unité d'évaluation selon l'une quelconque des revendications 15 à 23, **caractérisée en ce que** l'on indique, grâce au paramètre de perturbations, le stade auquel une maladie du métabolisme du glucose est probablement présent.

25. Système destiné à étudier le métabolisme du glucose d'un être humain concernant les particularités se rapportant à la maladie et/ou conditionnées par la maladie, comprenant :
une unité de mesure (1), grâce à laquelle la concentration de glucose g(t₁) à g(tn) d'un fluide corporel, en particulier le sang, est mesurée aux instants t₁ à tₙ, qui sont répartis sur une période d'au moins 4 heures, de préférence, d'au moins 6 heures, et une unité d'évaluation (3) selon l'une quelconque des revendications 14 à 24.

26. Système selon la revendication 25, **caractérisé en ce qu'**il comprend un dispositif d'affichage (5) destiné à afficher des points de données déterminés dans une représentation de l'espace de phase.

27. Système selon la revendication 25 ou 26, **caractérisé en ce que**, grâce au paramètre de perturbations, avec utilisation de données de thérapie sauvegardées dans une banque de données, on établit et on émet une recommandation thérapeutique.

28. Système selon l'une quelconque des revendications 25 à 27, **caractérisé en ce qu'**il comprend un implant ayant un réservoir d'insuline et une micropompe pour la libération contrôlée d'insuline dans la circulation sanguine d'un patient, un signal d'avertissement étant déclenché par l'unité d'évaluation (3) dès que le paramètre de perturbations s'écarte, à raison de plus d'une tolérance prédéterminée, d'une valeur de consigne prédéterminée.
